# EUROPÄISCHE PATENTANMELDUNG

(11) **EP 3 950 011 A1**
(43) Veröffentlichungstag der Anmeldung: **09.02.2022**
(21) Anmeldenummer: 21188816.9
(22) Anmeldetag: 30.07.2021
(51) Int. Cl.: A61L 2/10, A47K 10/48, A61L 2/00

(54) **HANDDESINFEKTIONSVORRICHTUNG UND VERFAHREN**

(30) Priorität: 31.07.2020 DE 102020120217
(71) Anmelder: LED3.0 GmbH, 41460 Neuss (DE)
(72) Erfinder: Elskamp, Dirk, 41472 Neuss (DE)
(74) Vertreter: Brinkmann & Partner

(57) **Zusammenfassung**

Die Erfindung betrifft eine Handdesinfektionsvorrichtung zur Handdesinfektion mittels UV-C Strahlung, mit einem Gehäuse, welches einen wenigstens einseitig offenen Volumenraum zur Aufnahme wenigstens einer zu desinfizierenden Hand aufweist, wobei im Gehäuse eine UV-C-LED angeordnet ist, mit welcher der Volumenraum mit UV-C-Strahlung bestrahlbar ist.

Ferner betrifft die Erfindung ein Verfahren zur Handdesinfektion durch UV-C Strahlung mittels einer Handdesinfektionsvorrichtung gemäß der Erfindung, bei dem wenigstens ein unbedeckter Hautabschnitt einer menschlichen Hand in dem Volumenraum zum Zwecke der Desinfektion zumindest zeitweise mit UV-C Strahlung bestrahlt wird, welche von der wenigstens einen UV-C-LED emittiert wird.

## Beschreibung

Die Erfindung betrifft eine Handdesinfektionsvorrichtung mittels UV-C Strahlung. Ferner betrifft die Erfindung ein Verfahren zur Handdesinfektion durch UV-C Strahlung mittels einer erfindungsgemäßen Handdesinfektionsvorrichtung.

Entkeimung, insbesondere Oberflächenentkeimung, mittels UV-Strahlung ist dem Grunde nach aus dem Stand der Technik bekannt. Dieser Anwendung liegt die Erkenntnis zu Grunde, dass Keime, insbesondere Bakterien und Viren, empfindlich gegenüber UV-Strahlung sind.

Gleichwohl ist die Entkeimung zur Handdesinfektion mittels UV-Strahlung bislang nicht wirtschaftlich sinnvoll nutzbar. Darüber hinaus ist es bei gängigen UV-Quellen, wie etwa Quecksilberdampflampen, Quarzlampen oder Schwarzlichtlampen nicht möglich die Strahlungsenergie so zu bündeln bzw. technisch anzuordnen, dass eine Wirksamkeit gegeben ist. Darüber hinaus sind diese wartungsintensiv und von vergleichsweise kurzer Lebensdauer.

Aus diesem Grund erfolgt die Handdesinfektion bzw. die Entkeimung zum aktuellen Zeitpunkt ausschließlich auf einer chemischen Basis und/oder unter Einsatz von antibakteriellen und/oder antiviralen Desinfektionsmitteln. Der Einsatz von antibakteriellen Desinfektionsmitteln fördert überdies die Entstehung multiresistenter Keime, die für Krankenhäuser und Pflegeheime zu einem immer größeren Problem werden.

Der Erfindung liegt damit die **Aufgabe** zugrunde, eine Handdesinfektionsvorrichtung und ein Verfahren anzugeben, welche die Entkeimung von Händen mittels UV-Strahlung ermöglicht und gegenüber bekannten Systemen eine erhöhte Wirksamkeit zu erreichen.

Zur **Lösung** der Aufgabe schlägt die Erfindung vorrichtungsseitig eine Handdesinfektionsvorrichtung zur Handdesinfektion mittels UV-C Strahlung, mit einem Gehäuse vor, welches Gehäuse einen wenigstens einseitig offenen Volumenraum zur Aufnahme wenigstens einer zu desinfizierenden Hand aufweist, **dadurch gekennzeichnet, dass** im Gehäuse eine UV-C-LED angeordnet ist, mit welcher der Volumenraum mit UV-C-Strahlung bestrahlbar ist.

Die Erfindung macht sich zwei miteinander synergierende Effekte zunutze. Zum einen hat es sich ergeben, dass UV-C Strahlung gegenüber Keimen, insbesondere gegenüber Viren, besonders wirksam ist. Der Ausdruck "UV-C Strahlung" bezeichnet im Sinne der Erfindung elektromagnetische Strahlung im ultravioletten Wellenlängenbereich von 280 bis 100 nm. Zum anderen ist der Einsatz einer LED als UV-C Strahlungsquelle hinsichtlich Energieverbrauch und Leistungsfähigkeit besonders vorteilhaft. Darüber hinaus weist eine im Besonderen als UV-C-LED ausgebildete LED eine gegenüber anderen UV-Quellen hohe Lebensdauer auf und bedarf vergleichsweise wenig Wartung. Der Begriff "LED" bezeichnet im Sinne der Erfindung eine Leuchtdiode. Der Begriff "UV-C-LED" bezeichnet im Sinne der Erfindung eine Leuchtdiode, die im Besonderen für die Emission von ultravioletter Strahlung im Wellenlängenbereich von 280 bis 100 nm ausgebildet ist. Durch die erfindungsgemäße Ausgestaltung ist es nun erstmals möglich, eine Entkeimung der Hände in einem Handtrockner / Gehäuse einzusetzen. Hierdurch kann vorzugsweise und mit besonderem Vorteil in Gänze auf entsprechende und/oder antibakterielle sowie antivirale Desinfektionsmittel verzichtet werden. Insbesondere die Handhabung, der Wartungsaufwand und die Benutzerfreundlichkeit der Handdesinfektion werden hierdurch verbessert. Darüber hinaus ist der Verzicht auf antibakterielle Desinfektionsmittel hinsichtlich der Bildung von multiresistenten Keimen von Vorteil.

Die Handdesinfektion kann hierbei vorzugsweise auf zwei unterschiedliche Weisen erfolgen:
1. Handdesinfektion integriert in einem Handtrockner:
   Durch das Einfügen der Hände in dem dafür vorgesehenen Schacht, erkennt der Bewegungs-; Erkennungssensor in der Vorrichtung die Veränderung. Automatisch startet der Handtrockner, eine Erkennungs-LED leuchtet und die UV-C-LED leuchtet ebenfalls. Nach Abschluss der Entkeimung leuchtet die Erkennungs-LED andersfarbig. Weiterhin besteht auch die Möglichkeit die Erkennungs-LED durch ein akustisches Signal zu ersetzen.
2. Handdesinfektion mit eigenem Gehäuse ohne Handtrockner:
   Durch das Einfügen der Hände in dem dafür vorgesehenen Schacht, erkennt der Bewegungs-; Erkennungssensor in der Vorrichtung die Veränderung. Automatisch leuchtet eine Erkennungs-LED und die UV-C-LED leuchtet ebenfalls. Nach Abschluss der Entkeimung leuchtet die Erkennungs-LED andersfarbig. Weiterhin besteht auch die Möglichkeit die Erkennungs-LED durch ein akustisches Signal zu ersetzen.

Gemäß einem bevorzugten Merkmal der Erfindung weist die erfindungsgemäße Vorrichtung eine Vielzahl von UV-C-LEDs auf. Gemäß einer ersten Alternative sind die UV-C-LEDs gleichmäßig innerhalb des Volumenraums verteilt angeordnet. Dies umfasst insbesondere die Anordnung von LEDs an der Seitenwand des Volumenraums, so dass die zu entkeimende Hand aus jeder Richtung homogen bestrahlbar ist. Ferner bevorzugt sind die UV-C-LEDs dabei über die gesamte Erstreckung der Seitenwand verteilt angeordnet. So wird sichergestellt, dass die zu entkeimende Hand während der gesamten Entkeimungssdauer bestrahlbar ist. Gemäß einer zweiten Alternative sind die UV-C-LEDs heterogen im Volumenraum angeordnet. Insbesondere ist vorgesehen, dass wenigstens ein Abschnitt der Seitenwand sämtliche UV-C-LEDs aufnimmt.

Vorzugsweise ist die UV-C-LED in Chipbauweise gefertigt. Sie verfügt dabei über eine Platine, auf welcher das hinsichtlich der zu emittierenden Strahlung wirksame Halbleiter-Bauelement angeordnet ist und die elektrischen Leitungen verlaufen. Besonders bevorzugt ist die UV-C-LED in SMD-Bauweise (surface mounted device) ausgebildet. Vorzugsweise weist der UV-C-LED-Chip dabei die Maße 3535 SMD oder 6060 SMD auf.

Gemäß einem bevorzugten Merkmal der Erfindung weist die UV-C-LED eine Leistung zwischen 35 mW und 1,5 W, vorzugsweise 500 mW bis 1,5 W, besonders bevorzugt 1 W bis 1,5 W, auf.

Gemäß einem weiteren bevorzugten Merkmal der Erfindung ist die UV-C-LED mit einer Linse ausgerüstet, welche zumindest das Halbleiter-Bauelement abdeckt. Dies dient zum einen dem Schutz vor Fremdeinwirkung und zum anderen der Bündelung der zu emittierenden Strahlung. Die Linse der UV-C-LED kann prinzipiell aus Kunststoff oder Glas gebildet sein. Hinsichtlich der bevorzugt vorgesehenen Bündelung der UV-Strahlung ist der Einsatz von Glas als Linsenmaterial bevorzugt. Dies deshalb, da Glas in der Regel bessere optische Eigenschaften aufweist. Darüber hinaus ist es bevorzugt vorgesehen, ein Glas mit einem vergleichsweise hohen Brechungsindex einzusetzen, um die Bündelung der Strahlung weiter zu präzisieren. Vorzugsweise wird ein Glas mit einem Brechungsindex von wenigstens 1,6 eingesetzt.

Gemäß einem bevorzugten Merkmal der Erfindung ist die UV-C-LED derart im Gehäuse angeordnet, dass deren Linse winklig zur Höhenrichtung des Volumenraums ausgerichtet ist. Es ist dabei vorgesehen, dass das Gehäuse oberseitig eine Öffnung bereitstellt, über welche der Volumenraum für die zu desinfizierende Hand zugänglich ist. Die Hand wird insofern bevorzugt von oben in den Volumenraum eingeführt. Die winklige, insbesondere rechtwinklige, Ausrichtung der Linse zur Höhenrichtung des Volumenraums bewirkt dabei eine verbesserte Strahlungsverteilung im Volumenraum. Ferner wird hierdurch sichergestellt, dass die emittierte UV-C-Strahlung die gesamten Handflächen erreicht. Zur weiteren Verbesserung der Wirksamkeit ist es vorgesehen, die emittierten Strahlen über die Linsenwirkung an sich hinaus zu bündeln. Hierzu weist die Linse der UV-C-LED bevorzugt eine zusätzliche Einfräsung zur weiteren Bündelung der zu emittierenden UV-C-Strahlung auf. Hinsichtlich der bevorzugt einzubringenden Einfräsung ist der Einsatz von Glas als Linsenmaterial bevorzugt. Die Einfräsung kann dabei mit größerer Präzision in Glas eingebracht werden.

Gemäß einem bevorzugten Merkmal der Erfindung ist vorgesehen, dass die LED mittels der dem Halbleiter-Bauelement abgewandten Seite der Platine an einer der Seitenwände des Gehäuses angeordnet ist. Vorzugsweise ist die LED dabei an einer sich parallel zum Gehäuse erstreckenden Seitenwand angeordnet. Hierdurch wird der Einstrahlungswinkel auf die zu entkeimende Hand seitens der LED verbessert.

Zur **Lösung** der Aufgabe schlägt die Erfindung ferner verfahrensseitig ein Verfahren zur Handdesinfektion durch UV-C Strahlung mittels einer Handdesinfektionsvorrichtung gemäß einem der Ansprüche 1 bis 9 vor, bei dem wenigstens ein unbedeckter Hautabschnitt einer menschlichen Hand in dem Volumenraum zum Zwecke der Desinfektion zumindest zeitweise mit UV-C Strahlung bestrahlt wird, welche von der wenigstens einen UV-C-LED emittiert wird.

Die Erfindung wird nachfolgend anhand eines für den Fachmann nicht beschränkend auszulegenden Ausführungsbeispiels im Detail erläutert. Dabei zeigen
- Fig.1: eine Handdesinfektionsvorrichtung gemäß der Erfindung in perspektivischer Darstellung;
- Fig.2: ein UV-C-LED-Board zur Anordnung in einer Handdesinfektionsvorrichtung gemäß Figur 1;
- Fig.3: ein optisches Signal-LED-Board zur Anordnung in einer Handdesinfektionsvorrichtung gemäß Figur 1.

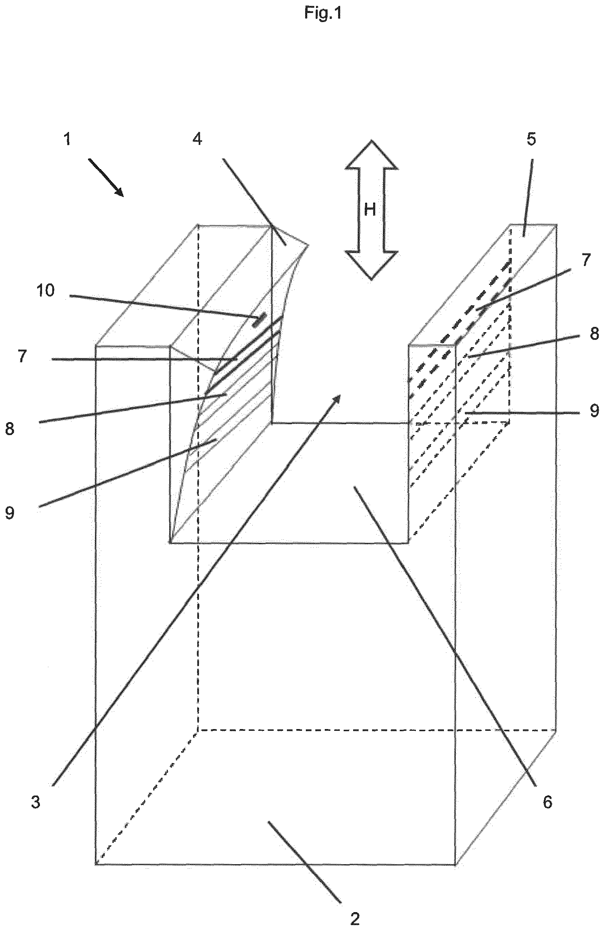

Figur 1 zeigt eine Handdesinfektionsvorrichtung 1 gemäß der Erfindung. Die Handdesinfektionsvorrichtung 1 weist ein Gehäuse 2 mit einem dreiseitig offenen Volumenraum 3. Der Volumenraum 3 wird durch zwei Seitenwände 4, 5 und einen Boden 6 des Gehäuses 2 räumlich begrenzt. Der Boden 6 verbindet die Seitenwände 4, 5 miteinander.

Die Seitenwände 4, 5 weisen vorliegend jeweils ein UV-C-LED-Board 7, eine schlitzförmige Lüftungsöffnung 8 für ein Trocknungsgebläse sowie ein optisches Signal-LED-Board 9 auf.

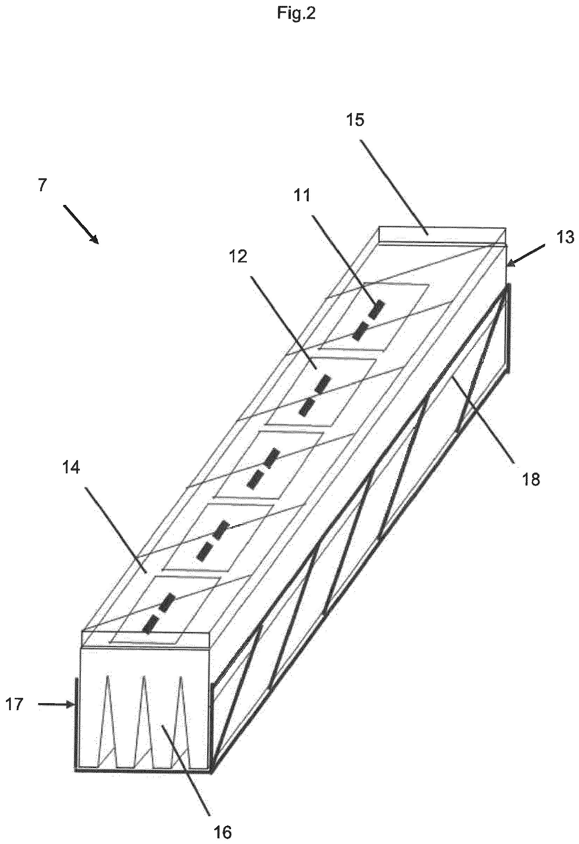

Das UV-C-LED-Board 7 dient der Aufnahme einer Mehrzahl vom UV-C-LEDs und ist im Detail in Figur 2 dargestellt. Das Board 7 ist leistenförmig ausgebildet und weist in Relation zu seiner Breite eine vergleichsweise große Länge auf. In Längsrichtung erstreckt sich das UV-C-LED-Board dabei im rechten Winkel zur Höhenrichtung H des Volumenraums 3.

Die Lüftungsöffnung 8 ist beabstandet und in Höhenrichtung unterhalb zu dem UV-C-LED-Board 7 in der Seitenwand 4, 5 angeordnet. Sie ist mit einer im Gehäuse 2 angeordneten, nicht dargestellten Trocknungseinrichtung strömungstechnisch verbunden. Die schlitzförmige Lüftungsöffnung 8 erstreckt sich in Längsrichtung im rechten Winkel zur Höhenrichtung H des Volumenraums 3 und damit parallel zum UV-C-LED-Board 7.

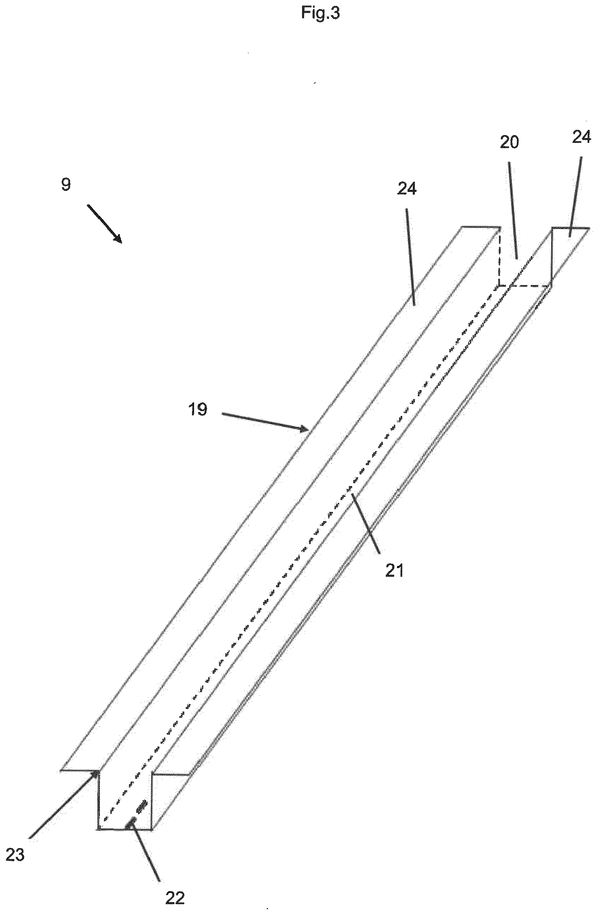

Das Signal-LED-Board 9 dient der Aufnahme einer Mehrzahl vom optischen LEDs und ist im Detail in Figur 3 dargestellt. Die optischen LEDs signalisieren dem Benutzer, den Status der Handdesinfektion. Insbesondere signalisieren sie, ob die UV-C-LEDs aktiv oder inaktiv sind. Das Board 9 ist leistenförmig ausgebildet und weist in Relation zu seiner Breite eine vergleichsweise große Länge auf. In Längsrichtung erstreckt sich das Signal-LED-Board 9 dabei im rechten Winkel zur Höhenrichtung H des Volumenraums 3. In Höhenrichtung H ist das LED-Board 9 jeweils beabstandet und unterhalb der Lüftungsöffnung 8 verlaufend in der Seitenwand 4, 5 angeordnet.

Sowohl das UV-C-LED-Board 7 als auch das Signal-LED-Board 9 sind derart an der Seitenwand 4, 5 angeordnet, dass die jeweilige UV-C- bzw. optische Strahlung in den Volumenraum 3 emittiert wird. Zu diesem Zweck sind die jeweiligen LEDs mit ihrer strahlungsaktiven Seite dem Volumenraum 3 zugewandt in dem jeweiligen Board 7, 9 angeordnet.

Zu erkennen ist ferner ein in der Seitenwand 4 angeordneter Sensor 10 zur Erkennung einer in den Volumenraum 3 eingeführten Hand. Der Sensor 10 kann dabei als Licht und/oder Wärmesensor ausgebildet sein. Der Sensor 10 ist mit einer im Gehäuse 2 angeordneten, nicht dargestellten Steuerungseinrichtung datentechnisch verbunden. Die Steuerungseinrichtung verfügt dabei über eine Rechnereinheit zur Auswertung der Sensordaten. Es ist dabei vorzugsweise vorgesehen, dass die UV-C-LEDs, die Trocknungseinrichtung und/oder die optischen Signal-LEDs in Abhängigkeit der ausgewerteten Sensordaten ansteuerbar sind. Zu diesem Zweck sind die die UV-C-LEDs, die Trocknungseinrichtung und/oder die optischen Signal-LEDs mit der Steuerungseinrichtung steuerungstechnisch verbunden. Hierdurch ist sichergestellt, dass der Desinfektionsvorgang nur dann gestartet wird, wenn auch tatsächlich eine menschliche Hand sensorisch detektiert wurde. Die Steuerungseinrichtung kann ferner über ein Zeitglied verfügen, mit welchem die Behandlungsdauer mittels UV-C-Strahlung und/oder Trocknungsluft zeitlich begrenzbar ist.

Die Seitenwand 4 ist vorliegend gewölbt ausgebildet. Hierdurch wird der UV-C-Einstrahlwinkel auf die zu desinfizierende Hand verbessert. Darüber hinaus ist der Abstand zwischen UV-C-LED zu der bestimmungsgemäß eingeführten Hand verringert, wodurch die Wirkung der Strahlung in vorteilhafter Weise verbessert wird

Figur 2 zeigt ein erfindungsgemäßes UV-C-LED-Board 7. Das UV-C-LED-Board 7 weist eine Mehrzahl von UV-C-LEDs 11 auf. Dabei sind jeweils zwei UV-C-LEDs 11 in ChipBauweise auf jeweils einer gemeinsamen Platine 12 angeordnet. Die Platinen 12 sind ihrerseits mit ihrer Rückseite auf einem Kühlkörper 13 angeordnet. Der Kühlkörper 13 stellt hierfür eine Kontaktfläche 14 bereit. Die UV-C-LEDs 11, die Platinen 12 sowie die Kontaktfläche 14 des Kühlkörpers 13 sind auf der strahlungsaktiven Seite des UV-C-LED-Boards 7 von einer durchgehenden Glasscheibe 15 überdeckt. Die Glasscheibe 15 dient als Linse mit einem Abstrahlwinkel zwischen 20° und 120°. Überdies schützt sie die sensiblen strahlungsaktiven Bauteile vor Verschmutzung und unsachgemäßer mechanischer Einwirkung.

Der Kühlkörper 13 ist als Strangpressprofil, vorzugsweise aus Aluminium oder einer Aluminiumlegierung, ausgebildet. Er weist auf seiner den LEDs 11 abgewandten Seite einen Wärmeübertragungsabschnitt 16 auf. Der Wärmeübertragungsabschnitt 16 verfügt über eine vergleichsweise große Oberfläche zur Abführung von beim Betrieb der LEDs 11 entstehender Wärme. Die Leistung der LEDs 11 wird hierdurch in vorteilhafter Weise verbessert. Der Kühlkörper 13 ist dabei in ein U-Profil 17 einsetzbar. Das U-Profil 17 ist in eine Vertiefung der Seitenwand 4, 5 des Gehäuses 2 seinerseits einsetzbar, so dass das UV-C-LED-Board 7 insgesamt in die Seitenwand 4, 5 eingelassen ist. Dies hat Vorteile hinsichtlich Wärmeabfuhr, mechanischem Schutz sowie der optischen Anmutung.

Der Kühlkörper 13 ist vorzugsweise mit dem U-Profil 17 formschlüssig verbindbar. Zu diesem Zweck weisen Kühlkörper 13 und/oder U-Profil 17 vorzugsweise entlang deren jeweiliger Längsseiten miteinander korrespondierende Verbindungselemente, insbesondere in Form einer Nut 18, auf. Die Verbindungselemente können in Form von miteinander korrespondierenden Einfräsungen gebildet sein.

Figur 3 zeigt ein Signal-LED-Board 9. Das Signal-LED-Board 9 weist ein Profil 19 auf. Das Profil 19 stellt eine im Querschnitt U-förmige Aufnahme 20 mit einem Boden 21 bereit. Der Boden 21 nimmt eine Mehrzahl von im sichtbaren Bereich des elektromagnetischen Spektrum emittierende optische LEDs 22 auf. Die LEDs 22 können so gewählt sein, dass sie farbiges Licht in einem beliebigen optischen Spektralbereich der Wellenlänge emittieren. Sie dienen der optischen Signalisierung des Behandlungsstatus.

Die Aufnahme 20 ist vorliegend vollständig, insbesondere mit ihrem Rand 23 bündig abschließend, mit einem transparenten Kunststoff, vorzugsweise Polyurethan oder einem darauf basierenden Copolymer, vergossen. Der daraus resultierende transparente Formkörper (nicht dargestellt) dient einerseits als Linse und andererseits dem Schutz der LEDs 22.

Das Profil 19 ist in eine in der Seitenwand 4, 5 ausgebildeten Vertiefung des Gehäuses 2 einsetzbar. Zur Befestigung des Profils 19 dienen in Längsrichtung des Profils 19 verlaufende und in Breitenrichtung des Profils 19 beidseitig ausgebildete Verbindungsstreifen 24. Der Verbindungsstreifen 24 ist vorzugsweise durch Umbiegen eines Randabschnitts des Profils 19 ausgebildet. Dies hat Vorteile hinsichtlich mechanischem Schutz sowie der optischen Anmutung.

### Bezugszeichenliste

- 1: Handdesinfektionsvorrichtung
- 2: Gehäuse
- 3: Volumenraum
- 4: Seitenwand
- 5: Seitenwand
- 6: Boden
- 7: UV-C-LED-Board
- 8: Lüftungsöffnung
- 9: optisches Signal-LED-Board
- 10: Sensor
- 11: UV-C-LED
- 12: Platine
- 13: Kühlkörper
- 14: Kontaktfläche
- 15: Glasscheibe
- 16: Wärmeübertragungsabschnitt
- 17: U-Profil
- 18: Nut
- 19: Profil
- 20: Aufnahme
- 21: Boden
- 22: optische LED
- 23: Rand
- 24: Verbindungsstreifen

## Patentansprüche

1. Handdesinfektionsvorrichtung zur Handdesinfektion mittels UV-C Strahlung, mit einem Gehäuse, welches einen wenigstens einseitig offenen Volumenraum zur Aufnahme wenigstens einer zu desinfizierenden Hand aufweist, **dadurch gekennzeichnet, dass** im Gehäuse eine UV-C-LED angeordnet ist, mit welcher der Volumenraum mit UV-C-Strahlung bestrahlbar ist.

2. Handdesinfektionsvorrichtung **gekennzeichnet durch** eine Trocknungseinrichtung zur Trocknung der zu desinfizierenden Hand mittels eines Luftstroms, wobei die Trocknungseinrichtung strömungstechnisch mit dem Volumenraum verbunden ist.

3. Handdesinfektionsvorrichtung nach Anspruch 1, **dadurch gekennzeichnet, dass** die UV-C-LED eine Linse aufweist, welche in winklig, insbesondere rechtwinklig, zur Höhenrichtung des Volumenraums ausgerichtet ist.

4. Handdesinfektionsvorrichtung **dadurch gekennzeichnet, dass** die Linse eine zusätzliche Einfräsung zur Bündelung der emittierten Strahlung aufweist.

5. Handdesinfektionsvorrichtung nach einem der Ansprüche 1 oder 2, **dadurch gekennzeichnet, dass** die wenigstens eine UV-C-LED in Chipbauweise ausgebildet ist und über eine Platine verfügt.

6. Handdesinfektionsvorrichtung nach Anspruch 3, **dadurch gekennzeichnet, dass** die UV-C-LED über ihre Platine an einer Seitenwand des Volumenraums angeordnet ist.

7. Handdesinfektionsvorrichtung nach einem der Ansprüche 3 oder 4, **dadurch gekennzeichnet, dass** die Platine mit einem Kühlkörper verbunden ist, welcher zwischen der Seitenwand des Gehäuses und der gehäuseseitigen Oberfläche der Platine angeordnet ist.

8. Handdesinfektionsvorrichtung nach einem der Ansprüche 1 bis 5, **dadurch gekennzeichnet, dass** das Gehäuse eine Breite, Höhe und/oder Länge von 15 cm bis 50 cm aufweist.

9. Handdesinfektionsvorrichtung, **dadurch gekennzeichnet, dass** eine Mehrzahl von UV-C-LEDs reihenförmig auf einem UV-C-LED-Board angeordnet ist, welches einen als Strangpressprofil ausgebildeten Kühlkörper aufweist.

10. Verfahren zur Handdesinfektion durch UV-C Strahlung mittels einer Handdesinfektionsvorrichtung gemäß einem der Ansprüche 1 bis 9, bei dem wenigstens ein unbedeckter Hautabschnitt einer menschlichen Hand in dem Volumenraum zum Zwecke der Desinfektion zumindest zeitweise mit UV-C Strahlung bestrahlt wird, welche von der wenigstens einen UV-C-LED emittiert wird.
